(19) Europäisches Patentamt
European Patent Office
Office européen des brevets

(11) EP 4 792 749 A1

(12) EUROPEAN PATENT APPLICATION

(43) Date of publication:
**19.08.2026 Bulletin 2026/34**

(21) Application number: **26157949.4**

(22) Date of filing: **11.02.2026**

(51) International Patent Classification (IPC):
***A61B 6/42*** *(2024.01)* ***A61B 6/46*** *(2024.01)*
***A61B 6/00*** *(2024.01)*

(52) Cooperative Patent Classification (CPC):
**A61B 6/4241; A61B 6/466; A61B 6/482; A61B 6/5205;** A61B 6/463; A61B 6/469; A61B 6/5294

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH LA MA MD TN**

(30) Priority: **14.02.2025 JP 2025022560**
**02.02.2026 JP 2026015173**

(71) Applicant: **Canon Medical Systems Corporation**
**Tochigi 324-0036 (JP)**

(72) Inventors:
- **NISHITAKA, Shinya**
**Otawara-shi, 324-0036 (JP)**
- **STROUD, Tyler**
**Otawara-shi, 324-0036 (JP)**

(74) Representative: **Marks & Clerk LLP**
**15 Fetter Lane**
**London EC4A 1BW (GB)**

# (54) IMAGE PROCESSING DEVICE, IMAGE PROCESSING METHOD, AND COMPUTER PROGRAM

(57) An image processing device (2) according to an embodiment includes processing circuitry (23) and a memory (22). The processing circuitry (23) displays first CT image data (90a) corresponding to a first keV value on a display (25) with a first window width and a first window level. The processing circuitry (23) determines a second window width and a second window level based on the first window width and level, respective CT values of a specific area corresponding to the first keV value and a second keV value, statistical information regarding the respective CT values of the specific area corresponding to the first and second keV values. The processing circuitry (23) displays second CT image data (90b) corresponding to the second keV value on the display (25) with the second window width and the second window level. An embodiment discloses an image processing method and a computer program.

FIG.2

1
X-RAY CT DEVICE
10
GANTRY DEVICE
40
CONSOLE DEVICE
11
16
17
13
14
P
33
12
DAS
18
Y
Z
X
CONTROL DEVICE
15
MEMORY
41
DISPLAY
42
INPUT INTERFACE
43
44
PROCESSING CIRCUITRY
CONTROL FUNCTION
441
PREPROCESSING FUNCTION
442
RECONSTRUCTION FUNCTION
443
IMAGE PROCESSING FUNCTION
444
TRANSMISSION FUNCTION
445
NETWORK INTERFACE
45
10
30
COUCH DEVICE
P
34
33
31
32
Y
Z
X



Processed by Luminess, 75001 PARIS (FR)

## Description

### FIELD

Embodiments described herein relate generally to an image processing device, an image processing method, and a computer program.

### BACKGROUND

Conventionally, X-ray computed tomography (CT) devices, especially dual energy (DE) CT devices and photon counting (PC) CT devices, is capable of acquiring X-ray energy information. Such DECT and PCCT devices are also capable of generating, from data acquired by scanning a subject, monochromatic image data (monochromatic X-ray image data) that depicts the subject with a single energy of any value. Therefore, such DECT and PCCT devices can acquire a plurality of pieces of monochromatic image data with different X-ray energy values for the same cross-section from data acquired in a single scan.

The contrast and the S/N ratio of images vary with energy values. Therefore, users may change the energy value (kilo electron volt: keV) of a display target when interpreting monochromatic image data to display monochromatic image data corresponding to other energy values. In such cases, if the window width (WW) and the window level (WL) remain the same as before the change, the visibility of the monochromatic image data corresponding to the changed energy value may be deteriorated.

### BRIEF DESCRIPTION OF THE DRAWINGS

FIG. 1 is a diagram illustrating an example of a medical image processing system according to a first embodiment;
FIG. 2 is a diagram illustrating an example of the configuration of an X-ray CT device according to the first embodiment;
FIG. 3 is a diagram illustrating an example of the configuration of a workstation according to the first embodiment;
FIG. 4 is a diagram illustrating an example of the configuration of a CT value database according to the first embodiment;
FIG. 5 is a diagram presenting an example of changes in the energy value of CT image data displayed on a display according to the first embodiment;
FIG. 6 is a diagram presenting an example of display of monochromatic image data according to a comparative example; and
FIG. 7 is a flowchart illustrating an example of a procedure of image display processing according to the first embodiment.

### DETAILED DESCRIPTION

Hereinafter, embodiments of an image processing device, an image processing method, and a computer program will be described in detail with reference to the accompanying drawings.

An image processing device according to an embodiment includes at least one piece of processing circuitry and at least one memory. The processing circuitry is configured to display first CT image data corresponding to a first keV value on a display with a first window width and a first window level. The processing circuitry is configured to determine a second window width and a second window level respectively different from the first window width and the first window level. The second window width and the second window level are determined based on the first window width, the first window level, CT values of a specific area corresponding to the first keV value, CT values of the specific area corresponding to a second keV value different from the first keV value, statistical information regarding the CT values of the specific area corresponding to the first keV value, and statistical information regarding the CT values of the specific area corresponding to the second keV value. The processing circuitry is configured to display second CT image data corresponding to the second keV value on the display with the second window width and the second window level.

### First Embodiment

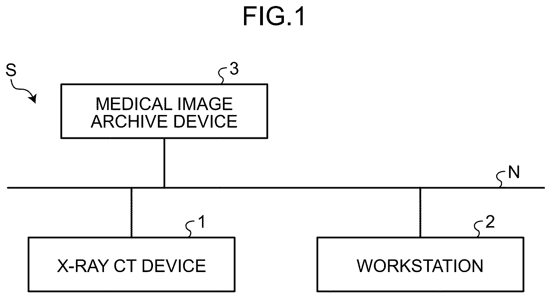

FIG. 1 is a diagram illustrating an example of a medical image processing system S according to a first embodiment. As illustrated in FIG. 1, the medical image processing system S includes, for example, an X-ray computed tomography (CT) device 1, a workstation 2, and a medical image archive device 3. The X-ray CT device 1, the workstation 2, and the medical image archive device 3 are connected to be communicable to each other via a network N such as a

hospital Local Area Network (LAN). In addition to each of the devices illustrated in FIG. 1, the medical image processing system S may include an electronic medical record system, a hospital information system (HIS), a laboratory information system (LIS), a radiology information system (RIS), and the like. The medical image processing system S may also be included as one of those systems, or may be communicatively connected to those systems.

The X-ray CT device 1 scans a subject with X-rays to acquire CT image data of the captured subject. In more detail, the X-ray CT device 1 according to the present embodiment is a dual energy (DE) CT device or a photon counting (PC) CT device capable of acquiring CT image data corresponding to a plurality of energy values. In the present embodiment, when simply referred to CT image data, it includes both multi-energy data corresponding to a plurality of energy values and monochromatic image data corresponding to a specific energy value.

The workstation 2 is a computer configured with a personal computer (PC), a server, or the like. The workstation 2 displays, for example, CT image data based on scans executed by the X-ray CT device 1 in various forms. The workstation 2 is an example of the image processing device according to the present embodiment.

The medical image archive device 3 is a device that saves medical image data acquired by capturing a subject. The medical image archive device 3 includes, for example, processing circuitry such as a processor, a memory, a network (NW) interface, an input interface, a display, and the like. More specifically, the medical image archive device 3 is, for example, a Picture Archiving and Communication System (PACS) server device that saves medical image data in a format compliant with Digital Imaging and Communications IN Medicine (DICOM). Medical image data is CT image data, for example.

The configuration of the X-ray CT device 1 will be described.

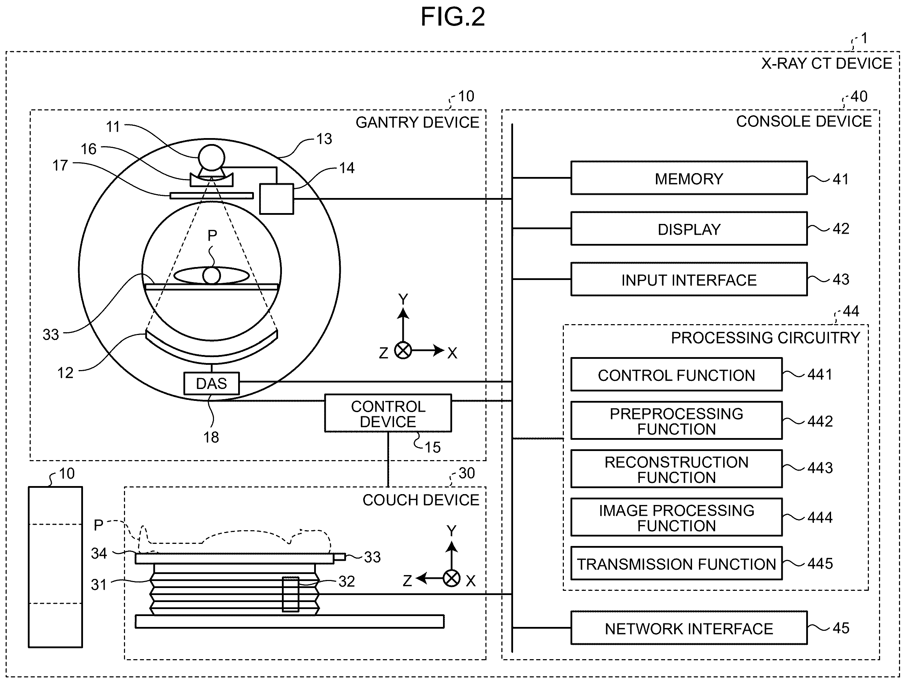

FIG. 2 is a diagram illustrating an example of the configuration of the X-ray CT device 1 according to the first embodiment. The X-ray CT device 1 according to the present embodiment is a DECT device capable of collecting detection data of X-rays corresponding to two or more kinds of energy values (tube voltages). The X-ray CT device 1 collects detection data of X-rays corresponding to at least two different energy values (tube voltages) that are 135 kilo volt peak (kVp) and 80 kVp.

As illustrated in FIG. 2, the X-ray CT device 1 includes a gantry device 10, a couch device 30, and a console device 40. In the present embodiment, the rotation axis of a rotation frame 13 or the longitudinal direction of a couchtop 33 of the couch device 30 in a non-tilted state is defined as the Z-axis direction, the axial direction orthogonal to the Z-axis direction and in parallel to the floor face as the X-axis direction, and the axial direction orthogonal to the Z-axis direction and perpendicular to the floor face as the Y-axis direction. Although plural gantry devices 10 are depicted in FIG. 2 for the sake of explanation, there is a single gantry device 10 in the configuration of the actual X-ray CT device 1.

The gantry device 10 and the couch device 30 operate according to operations performed by a user via the console device 40 or operations performed by the user via an operation unit provided in the gantry device 10 or the couch device 30. The gantry device 10, the couch device 30, and the console device 40 are connected with wire or wirelessly to be communicable to each other.

The gantry device 10 is a device with an imaging system that irradiates a subject P with X-rays and collects detection data of X-rays transmitted through the subject P. More specifically, the gantry device 10 includes an X-ray tube 11 (X-ray generation unit), a wedge 16, a collimator 17, an X-ray detector 12, an X-ray high voltage device 14, a data acquisition system (DAS) 18, the rotation frame 13, and a control device 15.

The X-ray tube 11 is a vacuum tube that generates X-rays by emitting thermal electrons from the cathode (filament) to the anode (target) through the application of high voltage and the supply of filament current from the X-ray high voltage device 14. X-rays are generated when the thermal electrons strike the target. The X-rays generated at the focus of the tube in the X-ray tube 11 are formed into a cone beam shape through the collimator 17, for example, and emitted to the subject P. The X-ray tube 11 includes, for example, a rotating anode X-ray tube that generates X-rays by irradiating the rotating anode with thermal electrons.

The X-ray tube 11 according to the present embodiment generates X-rays with two different voltage values, 135 kVp and 80 kVp. Examples of a method for generating X-rays with two different voltage values include Rapid kV Switching that switches between two kinds of high and low tube voltages at high speed in a single scan. Note that there are also other methods to be employed. In one example, the X-ray CT device 1 may include two X-ray tubes 11 that generate different tube voltages. Alternatively, a dual layer detector method may be employed, in which X-rays generated by the X-ray tube 11 are separated into two different energy values on the X-ray detector 12 side.

The X-ray detector 12 detects X-rays emitted from the X-ray tube 11 and outputs electrical signals corresponding to the detected X-ray dose to the DAS 18.

The rotation frame 13 supports the X-ray tube 11 and the X-ray detector 12 to be rotatable around the axis of rotation. In addition to the X-ray tube 11 and the X-ray detector 12, the rotation frame 13 further supports the X-ray high voltage device 14 and the DAS 18. Such a rotation frame 13 is housed in a substantially cylindrical casing with an opening (bore) that forms an imaging space.

The X-ray high voltage device 14 includes: a high voltage generation device that has electrical circuits such as a transformer and a rectifier, and has a function of generating high voltage to be applied to the X-ray tube 11 and filament

current to be supplied to the X-ray tube 11; and an X-ray control device that controls the output voltage according to the X-rays emitted by the X-ray tube 11. The high voltage generation device may be a transformer type or an inverter type. Note that the X-ray high voltage device 14 may be provided in the rotation frame 13 or may be provided on a fixed frame (not illustrated) side of the gantry device 10.

The control device 15 includes processing circuitry such as a central processing unit (CPU), and has a function of controlling the operations of the gantry device 10 and the couch device 30 upon receiving input signals from an input interface 43 installed at the console device 40 or the gantry device 10. For example, the control device 15 performs control to rotate the rotation frame 13, control to tilt the gantry device 10, and control to operate the couch device 30 and the couchtop 33 upon receiving input signals. Note that the control to tilt the gantry device 10 may be implemented by the control device 15 through rotating the rotation frame 13 around the axis parallel to the X-axis direction according to the tilt angle information input via the input interface 43 installed in the gantry device 10. The control device 15 may also be provided in the gantry device 10 or may be provided in the console device 40.

The wedge 16 is a filter for adjusting the X-ray dose of the X-rays emitted from the X-ray tube 11. Specifically, the wedge 16 is a filter that transmits and attenuates the X-rays emitted from the X-ray tube 11 such that the X-rays emitted from the X-ray tube 11 to the subject P come to be in a distribution determined in advance. The wedge 16 is, for example, a wedge filter or a bow-tie filter, which is a filter made of aluminum processed to be in a prescribed target angle and a prescribed thickness.

The collimator 17 is a lead plate or the like for narrowing down the X-rays transmitted through the wedge 16 to an X-ray irradiation range, and a slit is formed by combining a plurality of lead plates or the like.

The DAS 18 collects detection data regarding the subject P. In more detail, the DAS 18 collects signals detected by the X-ray detector 12. The DAS 18 includes, for example, an amplifier, an analog-to-digital (A/D) converter, and a control circuit. Note that the DAS 18 may further include other structural components. The DAS 18 also transmits (transfers) detection data (raw data) based on the X-ray detection result acquired from the X-ray detector 12 to the console device 40 under control of the control device 15. The DAS 18 may be connected to the console device 40 in a directly communicable manner or may be connected via the control device 15.

The couch device 30 is a device for placing and moving the subject P as a scan target, and it includes a base 31, a couch drive device 32, the couchtop 33, and a couchtop support frame 34. The base 31 is a casing that supports the couchtop support frame 34 to be movable in the vertical direction. The couch drive device 32 is a motor or an actuator that moves the couchtop 33 with the subject P placed thereon to the direction of the long axis of the couchtop 33. The couch drive device 32 moves the couchtop 33 according to the control of the console device 40 or the control of the control device 15. The couchtop 33 provided on the top face of the couchtop support frame 34 is a plate on which the subject P is placed. In addition to the couchtop 33, the couch drive device 32 may move the couchtop support frame 34 in the direction of the long axis of the couchtop 33.

The console device 40 is a device that executes control of the gantry device 10, generation of CT image data based on the scan results by the gantry device 10, and the like. The console device 40 includes a memory 41 (a storage unit), a display 42, the input interface 43 (an input unit), processing circuitry 44 (a processing unit), and a network interface 45 (a communication unit). Data communication among the memory 41, the display 42, the input interface 43, the processing circuitry 44, and the network interface 45 is performed via a bus.

The memory 41 is a storage device such as a hard disk drive (HDD), a solid state drive (SSD), or an integrated circuit storage device that stores various kinds of information. The memory 41 stores, for example, projection data and reconstructed image data. In addition to HDD, SSD, and the like, the memory 41 may also be a removable recording medium such as a compact disc (CD), a digital versatile disc (DVD), or a flash memory, or a drive device that reads and writes various kinds of information to/from a semiconductor memory element such as a random access memory (RAM). The save space of the memory 41 may be within the X-ray CT device 1 or may be in an external storage device connected via a network. The memory 41 also stores a control program of the console device 40 according to the present embodiment.

The display 42 displays various kinds of information. The display 42 outputs, for example, medical images (CT images) generated by the processing circuitry 44, a graphical user interface (GUI) for receiving various operations from the operator, and the like. Examples of the display 42 include a liquid crystal display (LCD), an organic electro luminescence display (OELD), a plasma display, etc. The display 42 may also be provided in the gantry device 10. The display 42 may be a desktop type or may be configured with a tablet terminal or the like capable of having wireless communication with the main body of the console device 40.

The input interface 43 receives various input operations from the operator, converts the received input operations into electrical signals, and outputs those to the processing circuitry 44. The input interface 43 receives, from the operator, a collection condition for collecting detection data, a reconstruction condition for reconstructing CT image data, an image processing condition for CT image data, and the like. Examples of the input interface 43 include a mouse, a keyboard, a trackball, switches, buttons, a joystick, a touchpad, a touch panel display, and the like.

In the present embodiment, the input interface 43 is not limited to those with physical operation components such

as a mouse, a keyboard, a trackball, switches, buttons, a joystick, a touchpad, a touch panel display, and the like. Processing circuitry that receives electrical signals corresponding to input operations from an external input device provided separately from the device and outputs those electrical signals to the processing circuitry 44 is also one example of the input interface 43. The input interface 43 is also an example of the input unit. Also, the input interface 43 may be provided in the gantry device 10. The input interface 43 may also be configured with a tablet terminal or the like capable of having wireless communication with the main body of the console device 40.

The network interface 45 is connected to the processing circuitry 44, and controls transmission and communication of various kinds of data to/from each of the devices connected via the network N. The network interface 45 may be implemented by a network card, a network adapter, a network interface controller (NIC), or the like.

The processing circuitry 44 is a processor that achieves functions corresponding to each of the programs by reading out the programs from the memory 41 and executing those. The processing circuitry 44 is provided with a control function 441, a preprocessing function 442, a reconstruction function 443, an image processing function 444, and a transmission function 445. The control function 441 is an example of a control unit. The preprocessing function 442 is an example of a preprocessing unit. The reconstruction function 443 is an example of a reconstruction unit. The image processing function 444 is an example of an image processing unit. The transmission function 445 is an example of a transmission unit.

The processing functions that are the control function 441, the preprocessing function 442, the reconstruction function 443, the image processing function 444, and the transmission function 445, which are structural components of the processing circuitry 44, are stored in the memory 41 in the form of programs that can be executed by a computer. In other words, the processing circuitry 44 with each program loaded thereon comes to have each of the functions illustrated within the processing circuitry 44 in FIG. 2. It is described in FIG. 2 that a single processor implements the processing functions performed by the control function 441, the preprocessing function 442, the reconstruction function 443, the image processing function 444, and the transmission function 445. However, it is also possible to configure the processing circuitry 44 by combining a plurality of independent processors, and each of the processors implements the function by executing the program. It is also described in FIG. 2 that a single memory 41 stores the programs corresponding to each of the processing functions. However, it is also possible to have a plurality of memories in a distributed manner, and the processing circuitry 44 reads the corresponding program from the individual memory.

While an example in which a "processor" reads and executes programs corresponding to respective functions from a memory is described in the above description, the embodiment is not limited thereto. The term "processor" refers to, for example, a circuit such as a central processing unit (CPU), a graphics processing unit (GPU), an application specific integrated circuit (ASIC), and a programmable logic device (for example, simple programmable logic device (SPLD), a complex programmable logic device (CPLD), and a field programmable gate array (FPGA)). When the processor is a CPU, for example, the processor executes the functions by reading out and executing the programs saved in the memory 41. On the other hand, when the processor is an ASIC, instead of saving the programs in the memory 41, the functions is incorporated directly into the circuitry of the processor as a logic circuits. Note that each of the processors according to the present embodiment is not limited to be configured as a single circuit per processor. It is also possible to combine a plurality of independent circuits to configure a single processor to implement the functions. Furthermore, it is also possible to integrate a plurality of structural components in FIG. 2 into a single processor and implement the functions.

The control function 441 controls various kinds of processing based on input operations received from the operator via the input interface 43. Specifically, the control function 441 controls the CT scans performed on the gantry device 10. The control function 441 controls collection processing of the detection data of X-rays passing through the subject P in the gantry device 10 by controlling the operations of the X-ray high voltage device 14, the X-ray detector 12, the control device 15, the DAS 18, and the couch drive device 32.

The preprocessing function 442 generates projection data by applying preprocessing such as logarithmic transformation processing, offset correction processing, sensitivity correction processing between channels, and beam hardening correction to the detection data output from the DAS 18.

The reconstruction function 443 generates CT image data by performing reconstruction processing using the filtered back projection method (FBP) or the like on the projection data generated by the preprocessing function 442. Since the X-ray CT device 1 according to the present embodiment is a DECT device capable of collecting detection data of X-rays corresponding to two different energy values (tube voltages), the reconstruction function 443 generates multi-energy data corresponding to a plurality of energy values by the reconstruction processing.

The reconstruction processing includes various kinds of processing, such as various kinds of correction processing like scattering correction and beam hardening correction, as well as application of reconstruction functions in the reconstruction condition. Note that the reconstruction processing executed by the reconstruction function 443 is not limited to the FBP method. It is also possible to use any known processing as appropriate, such as successive approximation reconstruction and a deep neural network that outputs a reconstructed image by inputting projection data. The reconstruction function 443 stores the reconstructed CT image data in the memory 41.

In the present embodiment, the data before preprocessing is referred to as raw data (detection data), the

detection data preprocessed by the preprocessing function 442 is referred to as projection data, and the projection data reconstructed by the reconstruction function 443 is referred to as CT image data.

The image processing function 444 converts the CT image data generated by the reconstruction function 443 into multiplanar tomographic image data or three-dimensional image data by a known method according to the input operations received from the operator via the input interface 43. The image processing function 444 may generate monochromatic image data from multi-energy data. Note that the three-dimensional image data and monochromatic image data may be generated by the reconstruction function 443. The image processing function 444 may also add ancillary information such as patient information of the subject P and examination date and time to the multi-energy data and monochromatic image data.

The transmission function 445 transmits CT image data to the workstation 2 and the medical image archive device 3 via the network interface 45 and the network N. In one example, the transmission function 445 transmits multi-energy data and monochromatic image data to the medical image archive device 3. Note that the CT image data may be transmitted to the medical image archive device 3 from the X-ray CT device 1 or from the workstation 2.

Next, the configuration of the workstation 2 will be described.

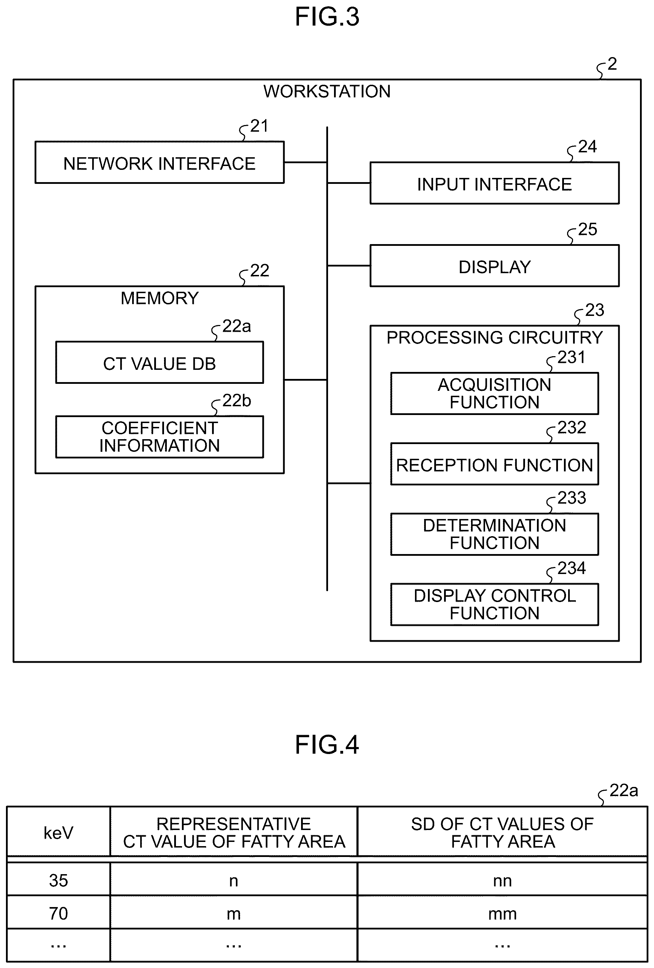

FIG. 3 is a diagram illustrating an example of the configuration of the workstation 2 according to the first embodiment. As illustrated in FIG. 3, the workstation 2 includes, for example, a network interface 21, a memory 22, processing circuitry 23, an input interface 24, and a display 25.

The network interface 21 receives CT image data that is transmitted from the X-ray CT device 1 via the network N. The network interface 21 may also receive CT image data from the medical image archive device 3. Note that the network interface 21 may acquire various kinds of information such as CT scan examination orders and imaging conditions from the X-ray CT device 1 or other systems.

The memory 22 stores various programs and various kinds of data used in the processing of the workstation 2. The memory 22 is implemented by, for example, a semiconductor memory element such as a read only memory (ROM), a RAM, or a flash memory, as well as a hard disk, an optical disk, or the like. Note that the memory 22 is also used as a hardware non-transitory recording medium.

The memory 22 also stores, for example, CT values of specific areas and statistical information regarding the CT values, which are associated with the energy values of the display targets. The CT values of specific areas stored in the memory 22 are, for example, representative values. Statistical information regarding the CT value is, for example, the standard deviation (SD), median, variance, and the like of the CT values. In the present embodiment, a case in which the statistical information regarding CT values is the standard deviation of the CT values will be described below as an example. The memory 22 stores a CT value database 22a in which representative values of the CT values of specific areas are registered in association with the standard deviations of the CT values. The memory 22 also stores coefficient information 22b.

FIG. 4 is a diagram illustrating an example of the configuration of the CT value database 22a according to the first embodiment. As illustrated in FIG. 4, the CT value database 22a is a database in which the energy values (keV) of the display target, the representative values of the CT values of the fatty area, and the standard deviations of the CT values of the fatty area are associated. The representative values of the CT values of specific areas and the standard deviations of the CT values of the specific areas registered in the CT value database 22a are the information used in the automatic changing processing of the window width and window level described later.

The specific area is an area that serves as the basis for the window width and the window level when displaying CT image data. In the automatic changing processing of the window width and window level described later, the window width and the window level with which a specific area in the CT image data can be displayed clearly are determined.

The specific area is, for example, soft tissue in the subject P. More specifically, in the present embodiment, the specific area is a fatty area, or an area where an organ designated by the user or the CT scan order is depicted. For example, in a case of an examination with a CT scan of the liver, the specific area may be the liver. Hereinafter, the present embodiment will be described by referring to a case as an example in which the specific area is a fatty area.

The energy values of the display target indicated in FIG. 4 are examples and are not limited to the values indicated in FIG. 4.

The representative value of the CT values of the fatty area registered in the CT value database 22a is the general CT value of the fatty area at each energy value. The CT value of the fatty area may be the average of the CT values of the fatty area in a plurality of pieces of CT image data generated in the past, or it may be a theoretical value.

The standard deviation of the CT values of the fatty area registered in the CT value database 22a is the standard deviation of the CT values of the fatty area at each energy value. The standard deviation of the CT values of the fatty area may be a value calculated from the CT values of the fatty area in a plurality of pieces of CT image data generated in the past, or it may be a theoretical value.

The coefficient information 22b is the values of coefficients $\alpha$ and $\beta$ used in the automatic changing processing of the window width and window level described later. The values of coefficients $\alpha$ and $\beta$ are defined in advance and stored in the memory 22. The coefficients $\alpha$ and $\beta$ will be discussed later.

The input interface 24 is implemented by, for example, a trackball, switch buttons, a mouse, a keyboard, and the like. Note that the input interface 24 in the present embodiment is not limited to only those with physical operation components such as a mouse, a keyboard, and the like. For example, electrical signal processing circuitry that receives electrical signals corresponding to input operations from an external input device provided separately from the workstation 2 and outputs those electrical signals to the processing circuitry 23 is also an example of the input interface 24.

The input interface 24 is connected to the processing circuitry 23, which converts various input operations received from the operator into electrical signals and outputs those to the processing circuitry 23.

The display 25 displays CT image data and various GUIs and the like under the control of processing circuitry 23.

The processing circuitry 23 is a processor that controls the entire workstation 2. The processing circuitry 23 includes an acquisition function 231, a reception function 232, a determination function 233, and a display control function 234. The acquisition function 231 is an example of an acquisition unit. The reception function 232 is an example of a reception unit. The determination function 233 is an example of a determination unit. The display control function 234 is an example of a display control unit.

The processing functions that are the acquisition function 231, the reception function 232, the determination function 233, and the display control function 234, which are structural components of the processing circuitry 23 are stored in the memory 22 in the form of programs that can be executed by a computer. In other words, the processing circuitry 23 with each program loaded thereon comes to have each of the functions illustrated within the processing circuitry 23 in FIG. 3. It is described in FIG. 3 that a single processor implements the processing functions performed by the acquisition function 231, the reception function 232, the determination function 233, and the display control function 234. However, it is also possible to configure the processing circuitry 23 by combining a plurality of independent processors, and each of the processors implements the function by executing the program. It is also described in FIG. 3 that a single memory 22 stores the programs corresponding to each of the processing functions. However, it is also possible to have a plurality of memories in a distributed manner, and the processing circuitry 23 reads the corresponding program from the individual memory.

While an example in which a "processor" reads and executes programs corresponding to respective functions from a memory is described in the above description, the embodiment is not limited thereto. The term "processor" corresponds to, for example, a circuit such as a CPU, a GPU, an ASIC, or a programmable logic device. When the processor is a CPU, for example, the processor executes the functions by reading out and executing the programs saved in the memory 22. In the meantime, when the processor is an ASIC, instead of saving the programs in the memory 22, the functions are incorporated directly into the circuitry of the processor as logic circuits. Note that each of the processors according to the present embodiment is not limited to be configured as a single circuit per processor. It is also possible to combine a plurality of independent circuits to configure a single processor to implement the functions. Furthermore, it is also possible to integrate a plurality of structural components in FIG. 3 into a single processor and implement the functions.

The acquisition function 231 acquires various kinds of data via the network interface 21. In more detail, the acquisition function 231 acquires CT image data from the X-ray CT device 1 or the medical image archive device 3. For example, the acquisition function 231 acquires multi-energy data corresponding to a plurality of energy values or acquires pieces of monochromatic image data each corresponding to a single energy value. The acquisition function 231 may also acquire various kinds of information such as CT scan examination orders and imaging conditions from the X-ray CT device 1 or other systems.

The reception function 232 receives various operations of the user via the input interface 24. For example, the reception function 232 receives an operation of the user to change the energy value of the CT image data to be displayed on the display 25.

In a case where the CT image data is monochromatic image data, the operation for changing the energy value of the CT image data as the display target refers to an operation for changing a state in which monochromatic image data with a certain energy value is being displayed on the display 25 to a state in which the monochromatic image data with another energy value is displayed on the display 25. At this time, the monochromatic image data of the energy value before change and the monochromatic image data of the other energy value after change indicate the same cross-sectional image data of the same subject P captured in a single scan. In the present embodiment, such an operation for changing the energy value of the CT image data as the display target is referred to as a changing operation. In other words, the changing operation is an operation to display the same cross-section of the same subject P with monochromatic image data corresponding to different energy values.

In one example, pieces of monochromatic image data of the subject P corresponding to different energy values captured in a single scan may be stored in the memory 22 as one set of monochromatic image data. Alternatively, multi-energy data capable of generating pieces of monochromatic image data corresponding to different energy values may be stored in the memory 22.

The determination function 233 determines the window width and window level for displaying the monochromatic image data corresponding to the changed energy value, upon receiving a changing operation from the user to change the energy value of the CT image data as the display target.

The display control function 234 displays various kinds of CT image data on the display 25. In more detail, the display control function 234 displays the monochromatic image data on the display 25 in the window width and the window level determined by the determination function 233.

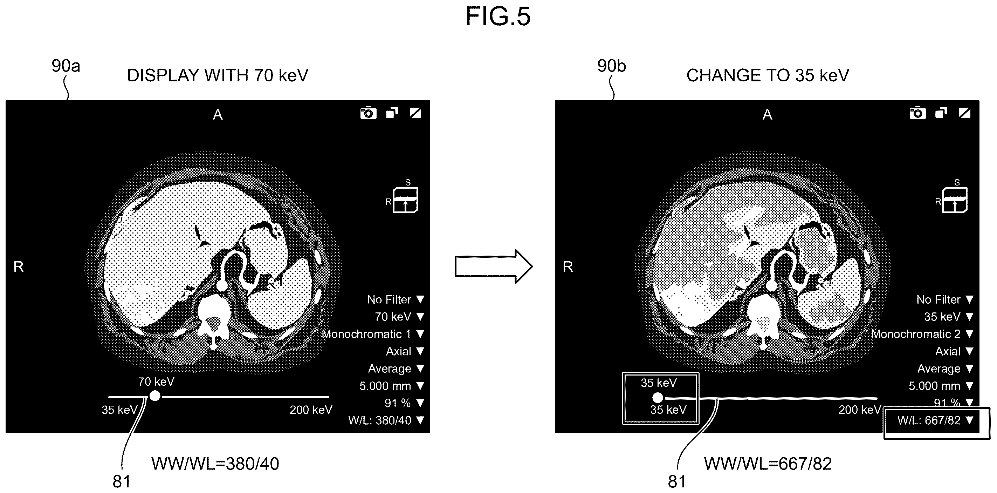

FIG. 5 is a diagram illustrating an example of changes in the energy value of the CT image data displayed on the display 25 according to the first embodiment. In the example illustrated in FIG. 5, the display control function 234 displays the monochromatic image data with a slide bar 81 superimposed thereon, which allows the user to change the energy values of the monochromatic image data as the display target. The operation for changing the energy value of the display target by operating the slide bar 81 is an example of the changing operation. Note that the changing operation is not limited to the operation of the slide bar 81, but may also be an input operation of an energy value, a selection operation from a list box, or the like.

In the example illustrated in FIG. 5, monochromatic image data 90a corresponding to an energy value of 70 keV is displayed. The display control function 234 displays the monochromatic image data 90a corresponding to the energy value of 70 keV with a window width of "380" and a window level of "40", for example. The window width and the window level of the monochromatic image data 90a may be values manually set by the user or may be values determined in advance, for example. As an example, initial values of the window width and the window level corresponding to each energy value may be stored in the memory 22, and the display control function 234 may first display the monochromatic image data 90a with the initial values of the window width and the window level. The user may also change the window width and the window level of the monochromatic image data 90a from the initial values by manual operations.

Note here that the user can operate the slide bar 81 to display monochromatic image data 90b corresponding to an energy value of 35 keV that is lower than 70 keV. The reception function 232 receives the energy value of 35 keV indicated on the slide bar 81 that is operated by the user, as the changed energy value. In other words, the reception function 232 receives the changing operation from the user to change the display target on the display 25 from the monochromatic image data 90a corresponding to the energy value of 70 keV to the monochromatic image data 90b corresponding to the energy value of 35 keV.

When the reception function 232 receives the changing operation from the user, the determination function 233 determines the window width and the window level suited for displaying the monochromatic image data 90b corresponding to the changed energy value based on the window width and the window level of the monochromatic image data 90a before the change, the representative values of the CT values in a specific area corresponding to the energy values before and after the change, and the standard deviations of the CT values of the specific area corresponding to the energy values before and after the change. In general, the appropriate window width and window level vary when the energy values of the display target are different.

In more detail, the determination function 233 determines the window width and the window level for displaying the monochromatic image data 90b corresponding to the energy value designated by the changing operation of the user, using the following Formula (1) and Formula (2). In Formula (1) and Formula (2), the window width (WW) and the window level (WL) of the monochromatic image data 90a displayed before the changing operation are referred to as "WW before change" and "WL before change". The window width and the window level suited for displaying the monochromatic image data 90b corresponding to the energy value designated by the changing operation of the user are referred to as "WW after change" and "WL after change".

WW after change = WW before change + (difference between the standard deviation of the CT values of a specific area corresponding to the energy value after change and the standard deviation of the CT values of the specific area corresponding to the energy value before change) $\times \alpha$ Formula (1):

WL after change = WL before change + (difference between the CT values of a specific area corresponding to the energy value after change and the CT values of the specific area corresponding to the energy value before change) $\times \beta$ Formula (2):

The determination function 233 reads and uses, from the CT value database 22a stored in the memory 22, "standard deviation of the CT values of the specific area corresponding to the energy value after change", "standard deviation of the CT values of the specific area corresponding to the energy value before change ", "CT values of the specific area corresponding to the energy value after change", and "CT values of the specific area corresponding to the energy value before change" in Formula (1) and Formula (2). The determination function 233 also reads and uses the coefficients $\alpha$ and $\beta$ in Formula (1) and Formula (2) from the coefficient information 22b stored in the memory 22.

Applying Formula (1) and Formula (2) to the example illustrated in FIG. 5, when the energy value of the monochromatic image data 90a displayed before the changing operation by the user is 70 keV and the energy value designated as the display target by the changing operation is 35 keV, the determination function 233 calculates the window width and the window level for displaying the monochromatic image data 90b corresponding to the energy value "35 keV"

designated through the changing operation by the user as in the following Formula (1a) and Formula (2a).

WW at 35 keV = WW at 70 keV + (difference between the standard deviation of the CT values of 35 keV fatty area and the standard deviation of the CT values of 70 keV fatty area) $\times \alpha$ Formula (1a):

: WL at 35 keV = WL at 70 keV + (difference between the CT values of 35 keV fatty area and the CT values of 70 keV fat) $\times \beta$ Formula (2a)

The monochromatic image data 90a in FIG. 5 is an example of first CT image data according to the present embodiment. The monochromatic image data 90b is an example of second CT image data according to the present embodiment. The energy value "70 keV" of the monochromatic image data 90a before change is an example of a first keV value. The energy value "35 keV" of the monochromatic image data 90b after change is an example of a second keV value. The window width "380" and the window level "40" of the monochromatic image data 90a before change are examples of a first window width and a first window level. The window width "667" and the window level "82" of the monochromatic image data 90b after change are examples of a second window width and a second window level.

When the reception function 232 receives a changing operation from the user, the display control function 234 displays the monochromatic image data 90b on the display 25 with the changed window width and the changed window level determined by the determination function 233.

The display control function 234 may generate and display on the display 25, the monochromatic image data 90a and 90b from the multi-energy data corresponding to a plurality of energy values in response to the operation of the user. Alternatively, the display control function 234 may read the monochromatic image data 90a and 90b generated in advance by corresponding to a single energy value from the memory 22 and display the data on the display 25 in response to the operation of the user. It is possible to employ known techniques for the format for storing the information on the multi-energy data or the monochromatic image data 90a, 90b in the memory 22 and for the method by which the display control function 234 reads out the information.

The monochromatic image data 90a and the monochromatic image data 90b are CT image data corresponding to different energy values, which are image data of the same cross-section of the same subject P captured in a single scan. For example, the monochromatic image data 90a and the monochromatic image data 90b are CT image data generated from a single piece of multi-energy data.

The user can change the target energy value and display the monochromatic image data 90a and 90b, which are images of an single energy value, by operating the slide bar 81. Hereinafter, the monochromatic image data 90a and 90b are simply referred to as the monochromatic image data 90, when there is no need to distinguish. The monochromatic image data 90b is also referred to as low-keV image data since it corresponds to a lower energy value than that of the monochromatic image data 90a.

In general, the monochromatic image data 90b corresponding to a low energy value (for example, 35 keV) can enhance the contrast of the depicted organs compared to the case of the monochromatic image data 90a corresponding to a standard energy value (for example, 70 keV). However, low-keV image data such as the monochromatic image data 90b has high CT values. Therefore, when the monochromatic image data 90b is displayed with the same window width and window level as the monochromatic image data 90a, the display on the display 25 may become too bright, which may deteriorate the visibility for the user.

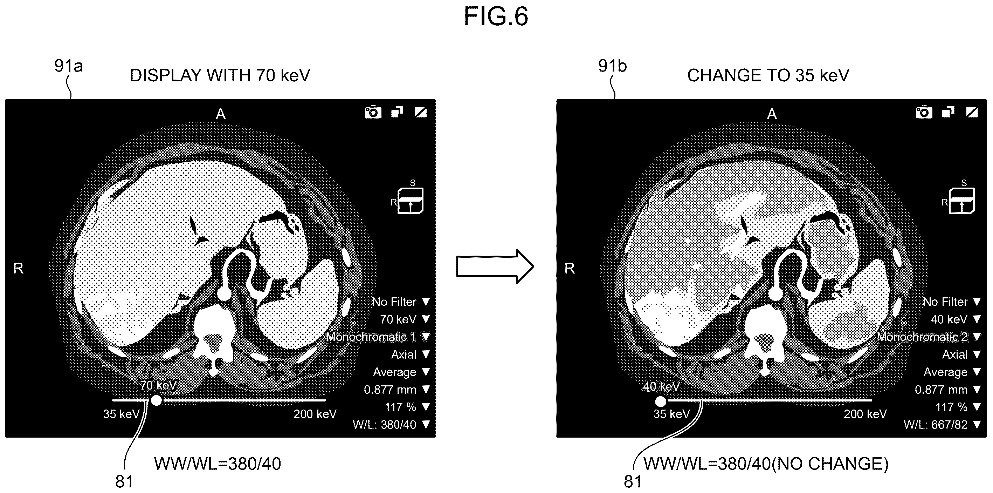

FIG. 6 is a diagram presenting an example of display of monochromatic image data 91a and 91b according to a comparative example. In the example in FIG. 6, the monochromatic image data 91a corresponds to an energy value of 70 keV. In the meantime, the monochromatic image data 91b corresponds to an energy value of 35 keV.

In FIG. 6, from a state where the monochromatic image data 91a is being displayed on the display 25 with the window width "380" and the window level "40", the energy value of the display target is changed to 35 keV by the user through a changing operation.

In FIG. 6, the 35 keV monochromatic image data 91b is displayed on the display 25 with the same window width "380" and window level "40" as before the change. Therefore, the display of the monochromatic image data 91b on the display 25 becomes brighter than the 70 keV monochromatic image data 91a before the change and the 35 keV monochromatic image data 90b in FIG. 5, which causes blown-out highlight in part of the area and deteriorates the visibility of the organs. In particular, soft tissues such as fatty areas are prone to cause blown-out highlight when low-keV image data is not displayed with appropriate window width and window level.

In contrast, in FIG. 5 described above, when the energy value of the display target is changed from 70 keV to 35 keV, the window width and the window level are changed to the values suited for displaying the 35 keV monochromatic image data 90b. Therefore, blown-out highlight is less recognized in the 35 keV monochromatic image data 90b presented

in FIG. 5 compared to the 35 keV monochromatic image data 91b presented in FIG. 6, and the visibility is maintained.

The image display processing will be described, which includes the automatic changing processing of the window width and window level executed by the workstation 2 configured as described above.

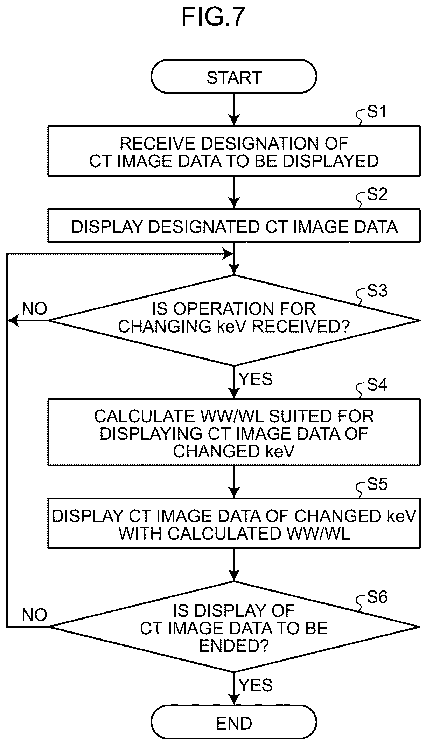

FIG. 7 is a flowchart illustrating an example of the procedure of the image display processing according to the first embodiment. Before execution of the flowchart, CT image data is acquired from the X-ray CT device 1 or the medical image archive device 3. CT image data may be multi-energy data corresponding to a plurality of energy values (for example, 35 keV, 70 keV) or may be single energy data corresponding to a plurality of tube voltage values.

The reception function 232 receives designation of CT image data as the display target from the user (S1). In one example, by input from the user such as patient information and examination date and time of the subject P, the reception function 232 may receive a designation of multi-energy data or a designation of a set of monochromatic image data 90 including the monochromatic image data 90a and 90b as the display target. Alternatively, the display control function 234 may display a selection screen with thumbnail images of the CT image data as the display target on the display 25 to allow the user to make a selection.

Upon receiving designation of CT image data as the display target from the user, the display control function 234 displays, on the display 25, the monochromatic image data 90 corresponding to a representative energy value among the selected CT image data (S2). Here, 70 keV is an example of a representative energy value. The representative energy values may be stored in the memory 22, for example, by being associated with multi-energy data or the monochromatic image data 90a and 90b, or may be designated in advance by the user. The display control function 234 displays the monochromatic image data 90a corresponding to 70 keV with the initial values of the window width and window level corresponding to 70 keV, among the multi-energy data selected by the user and one set of monochromatic image data 90.

Although omitted in FIG. 7, the user may operate the input interface 24 to change the window width and the window level for displaying the monochromatic image data 90a from the initial values as appropriate. When the reception function 232 receives an operation of the user to change the window width and the window level, the display control function 234 displays the monochromatic image data 90a with the changed window width and window level.

When the reception function 232 receives a changing operation from the user to change the energy value (keV) ("Yes" at S3), the determination function 233 calculates the window width and the window level suited for displaying the CT image data of the changed energy value (S4). Specifically, the determination function 233 uses Formula (1) and Formula (2) described above to calculate the window width and the window level suited for displaying the CT image data of the changed energy value (for example, the monochromatic image data 90b when the changed energy value is 35 keV). When the window width and the window level for displaying the monochromatic image data 90a are changed from the initial values by the user at the point of S3, the window width and the window level changed by the user are applied to the "WW before change" and "WL before change" used in Formula (1) and Formula (2).

The display control function 234 displays the CT image data of the changed energy value (for example, the monochromatic image data 90b) on the display 25 with the window width and the window level suited for displaying the CT image data of the changed energy value calculated by the determination function 233 (S5).

When an operation of the user to terminate the display of the CT image data is not received at the reception function 232 ("No" at S6), the processing returns to S3.

When an operation of the user to change the energy value (keV) is not received at the reception function 232 ("No" at S3), the reception function 232 waits for the operation of the user.

When an operation of the user to terminate the display of the CT image data is received at the reception function 232 ("Yes" at S6), the processing of this flowchart ends.

As described, the workstation 2 according to the present embodiment determines the window width and the window level suited for displaying the monochromatic image data 90b corresponding to the changed energy value based on the current window width and the current window level of the displayed monochromatic image data 90a, the CT values of a specific area corresponding to the energy values before and after the change, and the standard deviations of the CT values of the specific area corresponding to the energy values before and after the change, and displays the monochromatic image data 90b with the determined window width and window level. Therefore, with the workstation 2 according to the present embodiment, it is possible to display the monochromatic image data 90b with the appropriate window width and window level in accordance with the changes in the energy value of the display target.

As described above, since the contrast changes when the energy values of the display target are different, blown-out highlight or the like may occur when the monochromatic image data 90a and 90b corresponding to different energy values are displayed with the same window width and window level. Therefore, if the user manually adjusts the window width and the window level every time the energy value of the display target is changed while interpreting radiographs, it is time-consuming and interpretation work may be interrupted every time. In addition, in a case where the user has adjusted to the appropriate window width and window level for the 70 keV monochromatic image data 90a before being changed, if the 35 keV monochromatic image data 90b after being changed is to be displayed by resetting the adjusted current window width and window level, adjustment work needs to be performed every time, which goes to waste. With the workstation 2 according to the present embodiment, it is possible to reduce the time and effort as well as waste related to such work.

Moreover, upon receiving an operation of the user for changing the energy value of the display target during the display of the monochromatic image data 90a, the workstation 2 according to the present embodiment determines the window width and the window level suited for displaying the monochromatic image data 90b corresponding to the changed energy value, and displays the monochromatic image data 90b with the determined window width and window level. Therefore, with the workstation 2 according to the present embodiment, the user can change the energy value of the display target at a desired timing during the display of the monochromatic image data 90a, and view the monochromatic image data 90b after the change with the appropriate window width and window level.

In addition, the workstation 2 according to the present embodiment includes the memory 22 in which the CT value database 22a is stored. The workstation 2 according to the present embodiment determines the window width and the window level suited for displaying the monochromatic image data 90b corresponding to the changed energy value based on the representative values of the CT values of a specific area and the standard deviations of the CT values of the specific area with the energy values before and after change registered in the CT value database 22a. Therefore, with the workstation 2 according to the present embodiment, it is possible to reduce the computational load and time required for calculating the window width and the window level using the CT values and standard deviations stored in advance.

Furthermore, in the present embodiment, the specific area is a fatty area, or an area where an organ designated by the user or the examination order is depicted. Fatty areas are prone to have blown-out highlight and the like in low-keV data, and easily affected when the window width and the window level are not appropriate. In addition, by giving priority to the visibility of the organ designated by the user or the examination order, the accuracy of radiographic interpretation for that organ can be improved.

In the present embodiment, as the examples of pieces of CT image data corresponding to different energy values, the monochromatic image data 90a and 90b acquired from the same scan on the subject P are discussed. Since how the monochromatic image data 90 appears varies with the energy values, there is a need to display pieces of monochromatic image data 90a and 90b by changing the corresponding energy values with the appropriate window width and window level when the user interprets radiographs.

## Second Embodiment

In the first embodiment described above, the workstation 2 stores the CT value database 22a in the memory 22 in advance. In contrast, the workstation 2 according to a second embodiment dynamically determines the representative value of CT values of a specific area and the standard deviation of CT values of the specific area from the CT image data to be displayed.

The medical image processing system S according to the present embodiment includes the X-ray CT device 1, the workstation 2, and the medical image archive device 3 as in the first embodiment. The configurations of the X-ray CT device 1 and the medical image archive device 3 are the same as those of the first embodiment.

The workstation 2 according to the present embodiment includes the network interface 21, the memory 22, the processing circuitry 23, the input interface 24, and the display 25 as in the first embodiment.

The processing circuitry 23 of the workstation 2 according to the present embodiment includes the acquisition function 231, the reception function 232, the determination function 233, and the display control function 234 as in the first embodiment.

The acquisition function 231, the reception function 232, and the display control function 234 have the same functions as those of the first embodiment.

In addition to the functions same as those of the first embodiment, the determination function 233 according to the present embodiment determines the representative value of CT values of a specific area and the standard deviation of the CT values of the specific area from the monochromatic image data 90a and 90b. For example, the determination function 233 recognizes a specific area (for example, a fatty area or the like) from each of the monochromatic image data 90a and the monochromatic image data 90b by image processing. Then, the determination function 233 determines the representative value of the CT values of the specific area and the standard deviation of the CT values of the specific area corresponding to 70 keV from the monochromatic image data 90a corresponding to 70 keV. The determination function 233 also determines the representative value of the CT values of the specific area and the standard deviation of the CT values of the specific area corresponding to 35 keV from the monochromatic image data 90b corresponding to 35 keV.

Therefore, in addition to the effects same as those of the first embodiment, it is possible with the workstation 2 according to the present embodiment to display the monochromatic image data 90b with the appropriate window width and window level in accordance with the changes in the energy value of the display target even if the memory 22 does not have the CT value database 22a stored in advance.

Moreover, the workstation 2 according to the present embodiment determines the appropriate window width and window level for displaying the monochromatic image data 90b not based on the representative values and standard deviations of CT values stored in advance but based on the representatives value of the CT values of a specific area and

the standard deviation of the CT values of the specific area actually acquired from the monochromatic image data 90a and 90b as the display target. Therefore, it is possible to adjust the window width and the window level with high precision according to the actual CT values.

Third Embodiment

In the first embodiment described above, the values of the coefficients $\alpha$ and $\beta$ used for calculating the window width and the window level are prestored as the coefficient information 22b in the memory 22. In contrast, the workstation 2 according to a third embodiment outputs values of the coefficients $\alpha$ and $\beta$ by a first trained model.

The medical image processing system S according to the present embodiment includes the X-ray CT device 1, the workstation 2, and the medical image archive device 3 as in the first embodiment. The configurations of the X-ray CT device 1 and the medical image archive device 3 are the same as those of the first embodiment.

The workstation 2 according to the present embodiment includes the network interface 21, the memory 22, the processing circuitry 23, the input interface 24, and the display 25 as in the first embodiment.

The processing circuitry 23 of the workstation 2 according to the present embodiment includes the acquisition function 231, the reception function 232, the determination function 233, and the display control function 234 as in the first embodiment.

The acquisition function 231, the reception function 232, and the display control function 234 have the same functions as those of the first embodiment.

In addition to the functions same as those of the first embodiment, the determination function 233 according to the present embodiment causes the first trained model to output the coefficients $\alpha$ and $\beta$, and assigns the output coefficients $\alpha$ and $\beta$ to Formula (1) and Formula (2) described in the first embodiment to acquire the window width and the window level for displaying the monochromatic image data 90b corresponding to the energy value designated by a changing operation of the user.

The first trained model outputs the coefficient $\alpha$ for Formula (1) and the coefficient $\beta$ for Formula (2). The first trained model is an AI (Artificial Intelligence) model generated by deep learning or other machine learning. The first trained model is, for example, a model that has been trained by associating the energy values before change as well as the window width and the window level of the monochromatic image data 90a corresponding to such energy values with the energy values after change as well as the window width and the window level with which the monochromatic image data 90b corresponding to such energy values can be displayed appropriately, and the representative values of CT values of specific areas and the standard deviations of CT values of the specific areas of the monochromatic image data 90a and 90b before and after change. Note that the window width and the window level with which each piece of the monochromatic image data 90 can be displayed appropriately may be the window width and the window level manually adjusted by the user, for example. The training method and training data for the first trained model are not limited to those examples.

The determination function 233 may acquire the coefficients $\alpha$ and $\beta$ from the first trained model by inputting the energy value before change (for example, 70 keV), the energy value after change selected by the user (for example, 35 keV), and the window width and the window level of the monochromatic image data 90a before change into the first trained model.

As in the first embodiment, the determination function 233 acquires the information other than the coefficients $\alpha$ and $\beta$ to be assigned to Formula (1) and Formula (2) from the CT value database 22a stored in the memory 22 and the window width and the window level of the monochromatic image data 90a currently displayed on the display 25.

Note that the first trained model may be incorporated into the determination function 233. The first trained model may also be stored in the memory 22 and accessed by the determination function 233.

In addition to the effects same as those of the first embodiment, the workstation 2 according to the present embodiment having the first trained model that outputs the coefficient $\alpha$ for Formula (1) and the coefficient $\beta$ for Formula (2) may be able to determine more appropriate window width and window level by using the coefficient $\alpha$ and the coefficient $\beta$ stored in advance as fixed values. Even when, for example, the image quality of the monochromatic image data 90 changes and the required display mode changes due to the change in the imaging conditions or imaging technologies, the first trained model can be retrained to be optimized based on new training data.

Note that the workstation 2 according to the present embodiment may acquire the first trained model that has been trained externally, or the processing circuitry 23 of the workstation 2 may have a training function to train the first trained model. The training function is an example of a training unit.

Fourth Embodiment

In the third embodiment described above, the values of the coefficients $\alpha$ and $\beta$ used for calculating the window width and the window level are output by the first trained model. In contrast, in a fourth embodiment, the workstation 2 causes a second trained model to output the window width and the window level for displaying the monochromatic image

data 90b corresponding to the energy value designated by a changing operation of the user.

The medical image processing system S according to the present embodiment includes the X-ray CT device 1, the workstation 2, and the medical image archive device 3 as in the first embodiment. The configurations of the X-ray CT device 1 and the medical image archive device 3 are the same as those of the first embodiment.

The workstation 2 according to the present embodiment includes the network interface 21, the memory 22, the processing circuitry 23, the input interface 24, and the display 25 as in the first embodiment.

The processing circuitry 23 of the workstation 2 according to the present embodiment includes the acquisition function 231, the reception function 232, the determination function 233, and the display control function 234 as in the first embodiment.

The acquisition function 231, the reception function 232, and the display control function 234 have the same functions as those of the first embodiment.

In addition to the functions same as those of the first embodiment, the determination function 233 according to the present embodiment causes the second trained model to output the window width and the window level for displaying the monochromatic image data 90b corresponding to the energy value designated by a changing operation of the user.

Upon receiving input of the energy value before change as well as the window width and the window level suited for displaying the monochromatic image data 90a corresponding to such energy value, the representative values of the CT values of a specific area corresponding to the energy values before and after change, and the standard deviations of the CT values of the specific area corresponding to the energy values before and after change, the second trained model outputs the window width and the window level with which the monochromatic image data 90b corresponding to the energy value after change can be displayed appropriately.

The second trained model is an AI model generated by deep learning or other machine learning. The second trained model is, for example, a model that has been trained by associating the energy values before change as well as the window width and the window level of the monochromatic image data 90a corresponding to such energy values with the energy values after change as well as the window width and the window level with which the monochromatic image data 90b corresponding to such energy values can be displayed appropriately, and the representative values of CT values in specific areas and the standard deviations of CT values in the specific areas of the monochromatic image data 90a and 90b before and after change. The training method and training data for the second trained model are not limited to those examples.

In one example, the determination function 233 acquires the window width and the window level suited for displaying the monochromatic image data 90b corresponding to the energy value after change (for example, 35 keV) from the second trained model by inputting, to the second trained model, the energy value before change (for example, 70 keV), the changed energy value selected by the user (for example, 35 keV), and the window width and the window level of the monochromatic image data 90a before change.

In addition to the effects same as those of the first embodiment, it is possible with the workstation 2 according to the present embodiment to directly acquire the window width and the window level after change using the second trained model.

As in the third embodiment, the workstation 2 according to the present embodiment may acquire the second trained model that has been trained externally, or the processing circuitry 23 of the workstation 2 may have a training function to train the second trained model. The training function is an example of a training unit.

## Fifth Embodiment

In the first to fourth embodiments described above, the workstation 2 executes the image display processing including the automatic changing processing of the window width and window level. However, the processing may be executed by the X-ray CT device 1 or other devices. An example of other devices may be an information processing terminal such as a work PC of a radiologist, for example. An information processing terminal such as a work PC of a radiologist may be an example of the image processing device.

When the X-ray CT device 1 executes the image display processing including the automatic changing processing of the window width and window level, for example, the processing circuitry 44 of the X-ray CT device 1 may, in addition to the configuration illustrated in FIG. 2, further include the reception function 232, the determination function 233, and the display control function 234 among the functions described as the functions of the processing circuitry 23 of the workstation 2 in the first embodiment. In this case, the X-ray CT device 1 may be an example of the image processing device.

## Modification

While the monochromatic image data 90 is discussed as an example of CT image data to be displayed in each of the above-described embodiments, the CT image data to be displayed is not limited thereto. For example, the methods of determining the window width and the window level described in each of the above embodiments may be employed, when

displaying a plurality of pieces of CT image data acquired due to differences in the tube voltage using a single energy by an ordinary X-ray CT device other than a DECT device or a PCCT device.

Although each of the embodiments described above is targeted at reconstructed CT image data, the methods of determining the window width and the window level discussed in each of the above-described embodiments may be applied to projection data before reconstruction in order to determine the reconstruction function.

In addition, although each of the above embodiments is described by referring only to the changes in the energy values as an example, conditions other than energy values may be changed as well. For example, when any one of the image acquisition condition, the image generation condition, and the collection image information is changed, the determination function 233 of the workstation 2 may use the methods discussed in each of the embodiments to determine the window width and the window level suited for displaying the CT image data after the change in those conditions.

In addition, although each of the above-described embodiments refers to a case as an example where the display target is changed from the monochromatic image data 90a corresponding to a high energy value (for example, 70 keV) to the monochromatic image data 90b corresponding to a low energy value (for example, 35 keV), the methods of each of the embodiments described above can also be applied to a case where the display target is changed from the monochromatic image data 90b corresponding to a low energy value to the monochromatic image data 90a corresponding to a high energy value.

While 35 keV and 70 keV are discussed as examples of energy values in each of the above-described embodiments, the energy values are not limited thereto. In addition, the monochromatic image data 90 that can be displayed are not limited to two kinds.

Note that various kinds of data discussed herein are typically digital data.

According to at least one of the embodiments described above, it is possible to display X-ray CT image data with the appropriate window width and window level in accordance with the changes in the energy value of the display target.

While certain embodiments have been described, these embodiments have been presented by way of example only, and are not intended to limit the scope of the inventions. Indeed, the novel embodiments described herein may be embodied in a variety of other forms; furthermore, various omissions, substitutions and changes in the form of the embodiments described herein may be made without departing from the scope of the inventions. The accompanying claims are intended to cover such forms or modifications as would fall within the scope of the inventions.

In regard to the above-described embodiments, the following appendices are listed as aspects and selective features of the present disclosure.

Appendix 1

An X-ray computed tomography (CT) device, comprising:

an X-ray tube configured to emit X-rays to a subject;
an X-ray detector configured to detect the X-rays emitted from the X-ray tube;
at least one piece of processing circuitry; and
at least one memory, wherein
the processing circuitry is configured to

display first CT image data corresponding to a first kilo electron volt (keV) value on a display with a first window width and a first window level,
determine a second window width and a second window level respectively different from the first window width and the first window level, the second window width and the second window level being determined based on the first window width, the first window level, CT values of a specific area corresponding to the first keV value, CT values of the specific area corresponding to a second keV value different from the first keV value, statistical information regarding the CT values of the specific area corresponding to the first keV value, and statistical information regarding the CT values of the specific area corresponding to the second keV value, and
display second CT image data corresponding to the second keV value on the display with the second window width and the second window level.

Appendix 2

An image processing device, comprising:

at least one piece of processing circuitry; and
at least one memory, wherein
the processing circuitry is configured to

display first computed tomography (CT) image data corresponding to a first kilo electron volt (keV) value on a display with a first window width and a first window level,
determine a second window width and a second window level respectively different from the first window width and the first window level, the second window width and the second window level being determined based on the first window width, the first window level, CT values of a specific area corresponding to the first keV value, CT values of the specific area corresponding to a second keV value different from the first keV value, statistical information regarding the CT values of the specific area corresponding to the first keV value, and statistical information regarding the CT values of the specific area corresponding to the second keV value, and
display second CT image data corresponding to the second keV value on the display with the second window width and the second window level.

Appendix 3

The processing circuitry may be configured to, upon receiving a changing operation of a user for changing a display target from the first CT image data to the second CT image data,

determine the second window width and the second window level, and
display the second CT image data on the display with the second window width and the second window level.

Appendix 4

The memory may be configured to store a representative value of the CT values of the specific area corresponding to the first keV value, the statistical information regarding the CT values of the specific area corresponding to the first keV value, a representative value of the CT values of the specific area corresponding to the second keV value, and the statistical information regarding the CT values of the specific area corresponding to the second keV value, and
the processing circuitry may be configured to determine the second window width and the second window level based on information stored in the memory, the information including the representative value of the CT values of the specific area corresponding to the first keV value, the statistical information regarding the CT values of the specific area corresponding to the first keV value, the representative value of the CT values of the specific area corresponding to the second keV value, and the statistical information regarding the CT values of the specific area corresponding to the second keV value.

Appendix 5

The processing circuitry may be configured to

determine, from the first CT image data, a representative value of the CT values of the specific area corresponding to the first keV value and the statistical information regarding the CT values of the specific area corresponding to the first keV value, and
determine, from the second CT image data, a representative value of the CT values of the specific area corresponding to the second keV value and the statistical information regarding the CT values of the specific area corresponding to the second keV value.

Appendix 6

The specific area may be a fatty area, or an area where an organ designated by a user or an examination order is depicted.

Appendix 7

The first CT image data and the second CT image data may be pieces of monochromatic image data acquired by a same single scan performed on a subject.

Appendix 8

The statistical information regarding the CT values may be a standard deviation of the CT values.

Appendix 9

The processing circuitry may be configured to

calculate the second window width by a first formula defined by

second window width = first window width + (difference between standard deviation of CT values of specific area corresponding to second keV value and standard deviation of CT values of specific area corresponding to first keV value) $\times \alpha$, and

calculate the second window level by a second formula defined by

second window level = first window level + (difference between CT values of specific area corresponding to second keV value and CT values of specific area corresponding to first keV value) $\times \beta$.

Appendix 10

The memory may be configured to store a first trained model serving to output the coefficient $\alpha$ for the first formula and the coefficient $\beta$ for the second formula, and
the processing circuitry may be configured to

read the coefficient $\alpha$ and the coefficient $\beta$ output from the first trained model, and
assign the coefficient $\alpha$ and the coefficient $\beta$ respectively to the first formula and the second formula.

Appendix 11

The memory may be configured to store a second trained model serving to output the second window width and the second window level in response to input of the first window width, the first window level, the CT values of the specific area corresponding to the first keV value, the CT values of the specific area corresponding to the second keV value, the statistical information regarding the CT values of the specific area corresponding to the first keV value, and the statistical information regarding the CT values of the specific area corresponding to the second keV value, and
the processing circuitry may be configured to determine the second window width and the second window level by the second trained model.

Appendix 12

An image processing method implemented by a computer, the method comprising:

displaying first computed tomography (CT) image data corresponding to a first kilo electron volt (keV) value on a display with a first window width and a first window level;
determining a second window width and a second window level respectively different from the first window width and the first window level, the second window width and the second window level being determined based on the first window width, the first window level, CT values of a specific area corresponding to the first keV value, CT values of the specific area corresponding to a second keV value different from the first keV value, statistical information regarding the CT values of the specific area corresponding to the first keV value, and statistical information regarding the CT values of the specific area corresponding to the second keV value; and
displaying second CT image data corresponding to the second keV value on the display with the second window width and the second window level.

Appendix 13

A non-transitory computer readable recording medium on which programmed instructions are recorded, the programmed instructions causing a computer to execute:

displaying first computed tomography (CT) image data corresponding to a first kilo electron volt (keV) value on a display with a first window width and a first window level;
determining a second window width and a second window level respectively different from the first window width and

the first window level, the second window width and the second window level being determined based on the first window width, the first window level, CT values of a specific area corresponding to the first keV value, CT values of the specific area corresponding to a second keV value different from the first keV value, statistical information regarding the CT values of the specific area corresponding to the first keV value, and statistical information regarding the CT values of the specific area corresponding to the second keV value; and
displaying second CT image data corresponding to the second keV value on the display with the second window width and the second window level.

Appendix 14

A computer program comprising programmed instructions causing a computer to perform processing, the processing including:

displaying first computed tomography (CT) image data corresponding to a first kilo electron volt (keV) value on a display with a first window width and a first window level;
determining a second window width and a second window level respectively different from the first window width and the first window level, the second window width and the second window level being determined based on the first window width, the first window level, CT values of a specific area corresponding to the first keV value, CT values of the specific area corresponding to a second keV value different from the first keV value, statistical information regarding the CT values of the specific area corresponding to the first keV value, and statistical information regarding the CT values of the specific area corresponding to the second keV value; and
displaying second CT image data corresponding to the second keV value on the display with the second window width and the second window level.

## Claims

**1.** An image processing device (2), comprising:

at least one piece of processing circuitry (23); and
at least one memory (22), wherein
the processing circuitry (23) is configured to

display first computed tomography (CT) image data (90a) corresponding to a first kilo electron volt (keV) value on a display (25) with a first window width and a first window level,
determine a second window width and a second window level respectively different from the first window width and the first window level, the second window width and the second window level being determined based on the first window width, the first window level, CT values of a specific area corresponding to the first keV value, CT values of the specific area corresponding to a second keV value different from the first keV value, statistical information regarding the CT values of the specific area corresponding to the first keV value, and statistical information regarding the CT values of the specific area corresponding to the second keV value, and
display second CT image data (90b) corresponding to the second keV value on the display (25) with the second window width and the second window level.

**2.** The image processing device according to claim 1, wherein the processing circuitry (23) is configured to, upon receiving a changing operation of a user for changing a display target from the first CT image data to the second CT image data,

determine the second window width and the second window level, and
display the second CT image data on the display (25) with the second window width and the second window level.

**3.** The image processing device according to claim 1, wherein

the memory (22) is configured to store a representative value of the CT values of the specific area corresponding to the first keV value, the statistical information regarding the CT values of the specific area corresponding to the first keV value, a representative value of the CT values of the specific area corresponding to the second keV value, and the statistical information regarding the CT values of the specific area corresponding to the second keV value,

and

the processing circuitry (23) is configured to determine the second window width and the second window level based on information stored in the memory, the information including the representative value of the CT values of the specific area corresponding to the first keV value, the statistical information regarding the CT values of the specific area corresponding to the first keV value, the representative value of the CT values of the specific area corresponding to the second keV value, and the statistical information regarding the CT values of the specific area corresponding to the second keV value.

**4.** The image processing device according to claim 1, wherein the processing circuitry (23) is configured to

determine, from the first CT image data, a representative value of the CT values of the specific area corresponding to the first keV value and the statistical information regarding the CT values of the specific area corresponding to the first keV value, and

determine, from the second CT image data, a representative value of the CT values of the specific area corresponding to the second keV value and the statistical information regarding the CT values of the specific area corresponding to the second keV value.

**5.** The image processing device according to claim 1, wherein the specific area is a fatty area, or an area where an organ designated by a user or an examination order is depicted.

**6.** The image processing device according to claim 1, wherein the first CT image data and the second CT image data are pieces of monochromatic image data acquired by a same single scan performed on a subject.

**7.** The image processing device according to any one of claims 1 to 6, wherein the statistical information regarding the CT values is a standard deviation of the CT values.

**8.** The image processing device according to claim 7, wherein the processing circuitry (23) is configured to

calculate the second window width by a first formula defined by

second window width = first window width + (difference between standard deviation of CT values of specific area corresponding to second keV value and standard deviation of CT values of specific area corresponding to first keV value) $\times$ $\alpha$, and

calculate the second window level by a second formula defined by

second window level = first window level + (difference between CT values of specific area corresponding to second keV value and CT values of specific area corresponding to first keV value) $\times$ $\beta$.

**9.** The image processing device according to claim 8, wherein

the memory (22) is configured to store a first trained model serving to output the coefficient $\alpha$ for the first formula and the coefficient $\beta$ for the second formula, and

the processing circuitry (23) is configured to

read the coefficient $\alpha$ and the coefficient $\beta$ output from the first trained model, and

assign the coefficient $\alpha$ and the coefficient $\beta$ respectively to the first formula and the second formula.

**10.** The image processing device according to claim 1, wherein

the memory (22) is configured to store a second trained model serving to output the second window width and the second window level in response to input of the first window width, the first window level, the CT values of the specific area corresponding to the first keV value, the CT values of the specific area corresponding to the second keV value, the statistical information regarding the CT values of the specific area corresponding to the first keV value, and the statistical information regarding the CT values of the specific area corresponding to the second keV value, and

the processing circuitry (23) is configured to determine the second window width and the second window level by the second trained model.

**11.** An image processing method implemented by a computer (2), the method comprising:

displaying (S2) first computed tomography (CT) image data (90a) corresponding to a first kilo electron volt (keV) value on a display (25) with a first window width and a first window level;
determining (S4) a second window width and a second window level respectively different from the first window width and the first window level, the second window width and the second window level being determined based on the first window width, the first window level, CT values of a specific area corresponding to the first keV value, CT values of the specific area corresponding to a second keV value different from the first keV value, statistical information regarding the CT values of the specific area corresponding to the first keV value, and statistical information regarding the CT values of the specific area corresponding to the second keV value; and
displaying (S5) second CT image data (90b) corresponding to the second keV value on the display (25) with the second window width and the second window level.

**12.** A computer program comprising programmed instructions causing a computer to perform processing, the processing including:

displaying (S2) first computed tomography (CT) image data (90a) corresponding to a first kilo electron volt (keV) value on a display (25) with a first window width and a first window level;
determining (S4) a second window width and a second window level respectively different from the first window width and the first window level, the second window width and the second window level being determined based on the first window width, the first window level, CT values of a specific area corresponding to the first keV value, CT values of the specific area corresponding to a second keV value different from the first keV value, statistical information regarding the CT values of the specific area corresponding to the first keV value, and statistical information regarding the CT values of the specific area corresponding to the second keV value; and
displaying (S5) second CT image data (90b) corresponding to the second keV value on the display (25) with the second window width and the second window level.

FIG.1

S
3
MEDICAL IMAGE
ARCHIVE DEVICE
N
1
X-RAY CT DEVICE
2
WORKSTATION

FIG.2

1
X-RAY CT DEVICE
10
GANTRY DEVICE
40
CONSOLE DEVICE
11
16
17
13
14
P
33
12
DAS
18
Y
Z
X
CONTROL DEVICE
15
MEMORY
41
DISPLAY
42
INPUT INTERFACE
43
44
PROCESSING CIRCUITRY
CONTROL FUNCTION
441
PREPROCESSING FUNCTION
442
RECONSTRUCTION FUNCTION
443
IMAGE PROCESSING FUNCTION
444
TRANSMISSION FUNCTION
445
10
30
COUCH DEVICE
P
34
33
31
32
Y
Z
X
NETWORK INTERFACE
45

FIG.3

2
WORKSTATION
21
NETWORK INTERFACE
24
INPUT INTERFACE
25
DISPLAY
22
MEMORY
22a
CT VALUE DB
22b
COEFFICIENT INFORMATION
23
PROCESSING CIRCUITRY
231
ACQUISITION FUNCTION
232
RECEPTION FUNCTION
233
DETERMINATION FUNCTION
234
DISPLAY CONTROL FUNCTION

FIG.4

22a

| keV | REPRESENTATIVE CT VALUE OF FATTY AREA | SD OF CT VALUES OF FATTY AREA |
|---|---|---|
| 35 | n | nn |
| 70 | m | mm |
| ... | ... | ... |

FIG.5
DISPLAY WITH 70 keV
CHANGE TO 35 keV
90a
90b
A
R
S
No Filter ▼
70 keV ▼
Monochromatic 1 ▼
Axial ▼
Average ▼
5.000 mm ▼
91 % ▼
W/L: 380/40 ▼
35 keV
70 keV
200 keV
81
WW/WL=380/40
No Filter ▼
35 keV ▼
Monochromatic 2 ▼
Axial ▼
Average ▼
5.000 mm ▼
91 % ▼
W/L: 667/82 ▼
35 keV
35 keV
200 keV
81
WW/WL=667/82

FIG.6
DISPLAY WITH 70 keV
91a
A
R
S
No Filter ▼
70 keV ▼
Monochromatic 1 ▼
Axial ▼
Average ▼
0.877 mm ▼
117 % ▼
W/L: 380/40 ▼
70 keV
35 keV
200 keV
WW/WL=380/40
81
CHANGE TO 35 keV
91b
A
R
S
No Filter ▼
40 keV ▼
Monochromatic 2 ▼
Axial ▼
Average ▼
0.877 mm ▼
117 % ▼
W/L: 667/82 ▼
40 keV
35 keV
200 keV
WW/WL=380/40(NO CHANGE)
81

FIG.7

START
S1
RECEIVE DESIGNATION OF CT IMAGE DATA TO BE DISPLAYED
S2
DISPLAY DESIGNATED CT IMAGE DATA
S3
IS OPERATION FOR CHANGING keV RECEIVED?
NO
YES
S4
CALCULATE WW/WL SUITED FOR DISPLAYING CT IMAGE DATA OF CHANGED keV
S5
DISPLAY CT IMAGE DATA OF CHANGED keV WITH CALCULATED WW/WL
S6
IS DISPLAY OF CT IMAGE DATA TO BE ENDED?
NO
YES
END

Europäisches
Patentamt
European
Patent Office
Office européen
des brevets

# EUROPEAN SEARCH REPORT

Application Number
EP 26 15 7949

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X<br>A | EP 3 576 048 A1 (KONINKLIJKE PHILIPS NV [NL]) 4 December 2019 (2019-12-04)<br>* Equations (1) & (2); paragraphs [0007], [0076], [0058], [0041], [0044] - [0046], [0047], [0048], [0066], [0007] * | 1-7, 10-12<br>8,9 | INV.<br>A61B6/42<br>A61B6/46<br>A61B6/00 |
| A | US 2021/110583 A1 (LEE CHANG-LAE [KR] ET AL) 15 April 2021 (2021-04-15)<br>* paragraph [0157] * | 8,9 | |
| | | | TECHNICAL FIELDS SEARCHED (IPC) |
| | | | A61B |

The present search report has been drawn up for all claims

| Place of search | Date of completion of the search | Examiner |
|---|---|---|
| Munich | 29 June 2026 | Anscombe, Marcel |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

# ANNEX TO THE EUROPEAN SEARCH REPORT ON EUROPEAN PATENT APPLICATION NO. EP 26 15 7949

This annex lists the patent family members relating to the patent documents cited in the above-mentioned European search report. The members are as contained in the European Patent Office EDP file on
The European Patent Office is in no way liable for these particulars which are merely given for the purpose of information.

29-06-2026

| Patent document cited in search report | | Publication date | Patent family member(s) | | Publication date |
|---|---|---|---|---|---|
| EP 3576048 | A1 | 04-12-2019 | NONE | | |
| US 2021110583 | A1 | 15-04-2021 | EP 3644282 | A1 | 29-04-2020 |
| | | | KR 20190015084 | A | 13-02-2019 |
| | | | US 2021110583 | A1 | 15-04-2021 |

EPO FORM P0459

For more details about this annex : see Official Journal of the European Patent Office, No. 12/82